(19) Europäisches Patentamt
European Patent Office
Office européen des brevets

(11) Publication number : **0 495 823 B1**

(12) **EUROPEAN PATENT SPECIFICATION**

(45) Date of publication of patent specification : **13.04.94 Bulletin 94/15**

(51) Int. Cl.$^5$ : **C07D 301/00,** C07D 301/08, C07D 303/08

(21) Application number : **90914816.5**

(22) Date of filing : **11.10.90**

(86) International application number : **PCT/US90/05665**

(87) International publication number : **WO 91/05778 02.05.91 Gazette 91/10**

(54) **EPOXIDATION OF FLUORINE CONTAINING OLEFINS.**

(30) Priority : **12.10.89 US 420454**

(43) Date of publication of application : **29.07.92 Bulletin 92/31**

(45) Publication of the grant of the patent : **13.04.94 Bulletin 94/15**

(84) Designated Contracting States : **DE FR GB IT**

(56) References cited :
**FR-A- 2 529 890**
**ANGEW. CHEM. INT. ED. ENGL:, vol. 25, no. 6, 1986; S. ROZEN et al., pp. 554-555**
**FLUORINE CHEMISTRY REVIEWS, vol. 5, 1971, New York, NY (US); P. TARRANT et al., pp. 77-85**

(56) References cited :
**INDUSTRIAL AND ENGINEERING CHEMISTRY, vol. 49, no. 8, August 1957; D.D. SMITH et al., pp. 1241-1246**
**PATENT ABSTRACTS OF JAPAN, 14 November 1977; abstract**

(73) Proprietor : **E.I. DU PONT DE NEMOURS AND COMPANY**
**1007 Market Street**
**Wilmington Delaware 19898 (US)**

(72) Inventor : **ROZEN, Shlomo, M.**
**12 Barazani Street**
**69 121 Tel Aviv (IL)**
Inventor : **SMART, Bruce, Edmund**
**22 Beethoven Drive**
**Wilmington, DE 19807 (US)**

(74) Representative : **Jones, Alan John et al**
**CARPMAELS & RANSFORD 43 Bloomsbury Square**
**London, WC1A 2RA (GB)**

**EP 0 495 823 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to the synthesis of epoxides from fluorine-containing olefins using elemental fluorine in mixtures of water and acetonitrile.

### BACKGROUND OF THE INVENTION

The epoxidation of olefins is a well known process practiced on a large industrial scale, for example see the Kirk-Othmer Encyclopedia of Chemical Technology, 3rd Ed., vol. 9, John Wiley & Sons, New York, 1980, p. 251-266. The most common method of epoxidation is the direct epoxidation of the olefin with an oxidizing agent such as a peracid, hydrogen peroxide or hypochlorous acid.

However, this method is usually not applicable to fluorinated olefins; other methods, which usually require more than one step, can be used to produce fluorine containing epoxides. These are reviewed by P. Tarrant, et al., in Fluorine Chemistry Reviews, vol. 5, Marcel Dekker, Inc., New York, 1971, p. 77-85, and specific examples are found in French Patent 2,529,890A, Japanese Patent 52-136,107 and USSR Patent No. 390,084. It is believed that the fluorinated olefins do not form epoxides in simple epoxidation reactions usually applicable to most olefins because of the electron deficient nature of the olefinic bond, caused by the presence of the fluorine atoms. This inability to undergo simple epoxidation reactions is illustrated hereinafter in Example 6.

S. Rozen and M. Brand, Angew. Chem. Int. Ed. Engl., vol. 25, 554-555 (1986) describe the direct epoxidation of olefins using a mixture of (elemental) fluorine, water and acetonitrile. The utility of this reaction with fluorinated olefins is neither discussed nor disclosed.

Fluorine containing epoxides are of special interest in polymer technology as they give rise to polymers with desirable properties. For example, D. D. Smith, et al., Ind. Eng. Chem., vol. 49, pg. 1241-1246 (1957), report such polymers are excellent low load lubricants. Thus a need exists for a direct efficient epoxidation process for fluorine containing olefins.

It is therefore an object of the present invention to provide a process for the direct epoxidation of fluorine containing olefins.

### SUMMARY OF THE INVENTION

The present invention comprises a process for direct epoxidation of fluorinated olefins wherein first an oxidizing reagent is created by passing elemental fluorine through a mixture of acetonitrile and water, and then a fluorine containing olefin is contacted with the oxidizing solution to yield the desired epoxide.

### DETAILED DESCRIPTION OF THE INVENTION

When diluted fluorine is passed through a cold mixture of acetonitrile-water an oxidizing reagent, stable at temperatures of up to 25°C for several hours, is formed. Unlike other direct epoxidation methods, this oxidizing reagent can be used to epoxidize the much more inert and resistant polyfluorinated olefins.

Fluorine is of course a strong oxidizer and a very corrosive material. An appropriate vacuum line made from copper or monel in a well ventilated area should be constructed for working with this element. The epoxidation reactions themselves can be carried out in glass vessels.

Fluorinated olefins suitable for use in the epoxidation process of the present invention include those of formula (I) and (II) as follows:

$$ZCH=CH_2 \qquad (I)$$

$$YCH_2CH=CH_2 \qquad (II)$$

wherein

Z is $ACF_2$-, or perfluoroaryl;

Y is

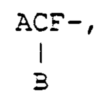

or perfluoroaryl;

A is perfluoroalkyl, fluorine, hydrocarbyl, or substituted hydrocarbyl; and

B is fluorine or perfluoroalkyl.

Suitable substituents when A is substituted hydrocarbyl include the vinyl group, $CH_2$=CH-, and any substituent inert under the reaction conditions of the process and which does not interfere with the process. When the vinyl group, $H_2C$=CH-, is present it too will be epoxidized, assuming enough oxidizing reagent is used. Inert substituents also include groups between hydrocarbyl segments such as ethers. Examples of suitable substituents include chlorine, fluorine, esters, ethers, and ketones.

Preferred fluorine containing olefins for use herein include those of formula (I) wherein Z is $ACF_2$-, or perfluoroaryl, and A is perfluorohydrocarbyl and those of formula (II) wherein Y is

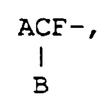

or perfluoroaryl, A is perfluorohydrocarbyl, and B is fluorine. Olefins of the formula (I) wherein Z is $ACF_2$ and A is perfluoro-n-alkyl or of formula (II) wherein Y is

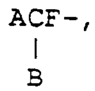

A is perfluoro-n-alkyl and B is fluorine are most preferred. In another preferred embodiment, Z is $ACF_2$-, and A is $CH_2=CH(CF_2)_n$-, wherein n is an integer from 0 to about 30.

Fluorinated olefins useful in the present process include, but are not limited to (perfluoropropyl)ethylene, 3,3,3-trifluoropropene, (perfluorobutyl)ethylene, (perfluorohexyl)ethylene, 9,10-dichloro-3,3,4, 4,6,7,7,9,10,10-decafluoro-5-trifluoromethyl-5,8-dioxa-1-decene, pentafluorophenylethylene, 3-(perfluorohexyl)propene, 3-(perfluorooctyl)propene, 3-(perfluorooctadecyl)propene, and 3,3-difluorocyclohexene, 3-pentafluorophenylpropane and 3,3,4,4,5,5,6, 6,7,7,8,8-dodecafluoro-1,9-decene.

The reaction proceeds according to the following equations:

A. $F_2 + H_2O/CH_3CN \rightarrow F_2/H_2O/CH_3CN$

B1. $ZCH=CH_2$ (I) + $F_2/H_2O/CH_3CN \rightarrow$

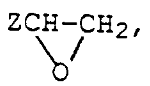

or

B2. $YCH_2CH=CH_2$ (II) + $F_2/H_2O/CH_3CN \rightarrow$

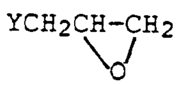

A suitable temperature range for the process of the present invention is from about -15°C to about 30°C. Preferably the reaction is conducted at from about 0°C to about 25°C. Reaction times can range from about 1 minute to 3 or more hours. A typical reaction time is from about 2 to 3 hours. The desired product may be isolated via extraction followed by distillation or evaporation of the solvent.

Mixtures of up to 25%, preferably 10-15% fluorine diluted with an inert gas such as nitrogen are used in the preparation of the oxidizing reagent. The gas mixtures are usually prepared in a secondary container before passing into the water/acetonitrile mixture. The gas mixture is then passed at a rate of about 400 ml per minute through a cold (-10°C) and vigorously stirred mixture of acetonitrile and water. The ratio of acetonitrile to water is about 10:1, for example 400 ml of acetonitrile and 40 ml of water. The formation of the oxidizing reagent can be monitored by reacting aliquots with acidic aqueous solution of potassium iodide. The liberated iodine is then titrated with thiosulfate. Concentrations of more than one mol/liter of oxidizing reagent have been obtained.

The oxidizing reagent is then contacted with a suitable fluorinated olefin to obtain the desired epoxide. An appropriate amount of olefin is dissolved in solvent such as methylene chloride, chloroform, or a fluorocarbon, cooled to about 0°C, and added in one portion to the reaction vessel containing the oxidizing agent. The solvent should be inert under process conditions. For example, most unsaturated solvents, such as hexene and toluene, react with the oxidizing reagent, and should be avoided. A 6-10 fold excess of the oxidizing reagent is employed. Except in the case of the more reactive olefins, the cooling bath is removed and the reaction stopped after about 3 hours by neutralizing it with saturated sodium bicarbonate solution. It should be noted that the reaction could be conducted much longer, but since most of the oxidizing reagent is decomposed after 2 to 3 hours, little additional product is obtained. A yield of about 70-80% of the epoxide can be obtained in about 80% conversion. The reaction mixture can then be poured into water, extracted with an organic solvent such as $CFCl_3$, and neutralized, usually by washing with sodium bicarbonate and then water. The organic layer is dried, typically over anhydrous $MgSO_4$, and the solvent distilled, preferably at atmospheric pressure. The crude product is usually distilled under reduced pressure.

The following Examples 1-5 demonstrate the process of the present invention, but are not intended to limit it in any manner. The following Example 6 demonstrates that fluorine containing olefins do not undergo the typical simple epoxidation reactions known in the art.

## EXAMPLE 1

Epoxidation of (Perfluorobutyl)ethylene Oxidizing solution containing 250 mmol of oxidant was made by preparing a solution of 400 ml of $CH_3CN$ and 40 ml of $H_2O$ which was then cooled to -10°C. Then 10% fluorine in nitrogen was bubbled through the hollow shaft of a vibromixer equipped with a stirring disk (from Chemap AG), which causes vigorous stirring and the formation of fine gas bubbles. Aliquots were periodically withdrawn and titrated as previously described for oxidizing reagent. A final total of 360 mmoles of oxidizing agent was formed. To this solution 15 g (61 mmol) of (perfluorobutyl)ethylene dissolved in 30 ml $CH_2Cl_2$ was added. The reaction was left overnight and poured into 1.5 l of water and extracted 3 times with 200 ml of $CFCl_2$. The organic layer was then neutralized with sodium bicarbonate solution, washed with water and dried over anhydrous $MgSO_4$. The organic solvent was removed by distillation through an efficient distillation column. The epoxide was then distilled to give 3 g of the starting material and about 10 g of the epoxide; b.p. = 81-83°C; IR = 1240 $cm^{-1}$; $^1H$ NMR = 3.0 (2H, d of narrow m, J = 10 Hz), 3.5 ppm (1H, t of narrow m, J = 10 Hz), $^{19}F$

NMR = -81.6 ppm (3F, t of narrow m, J = 10 Hz); MS m/e = 262 (M+).

EXAMPLE 2

Epoxidation of (Perfluorohexyl)ethylene

The oxidizing reagent (230 mmol) was prepared in 440 ml of $CH_3CN/H_2O$ solution (10:1) as in Example 1. Then 20 g of (perfluorohexyl)ethylene was dissolved in 20 ml of $CH_2Cl_2$ and added to the oxidizing reagent solution. After 2 h the reaction was neutralized with bicarbonate. Then 230 ml of the reaction mixture was distilled at 80 mm. This cut consisted mainly of acetonitrile and the desired epoxide. It was poured into 1 liter water and worked up as above in Example 1. The epoxide (80% yield at 60% conversion) was distilled at 20-22°C at 1 mm. IR = 1200, 1240 $cm^{-1}$; $^1H$ NMR = 3.0 (2H, d of narrow m, J = 10 Hz), 3.5 ppm (1H, t of narrow m, J = 10 Hz); $^{19}F$ NMR = -81.4 ppm (3F, t of narrow m, J = 10 Hz), MS m/e = 362 (M+).

EXAMPLE 3

Epoxidation of (Pentafluorophenyl)ethylene

The oxidizing reagent (100 mmol) was prepared in 440 ml of $CH_3CN/H_2O$ solution (10:1) as in Example 1. The 8 g of (pentafluorophenyl)ethylene was dissolved in 20 ml of $CH_2Cl_2$ and added to the reagent solution. After 5 min the reaction was neutralized with bicarbonate. Most of the liquids were distilled under reduced pressure (80 mm). The remaining liquid (50 ml) was poured into water, extracted with $CFCl_3$ and worked-up as in Example 1. The epoxide was distilled at 34-37°C at 0.1 # mm; yield 6.7 g (85%). $^1H$ NMR = 3.2 (2H, 2 narrow m), 4.0 ppm (1H, narrow m); $^{19}F$ NMR = -143.9 (2F, m), -154.1 (1F, t, J = 21 Hz), -162.5 ppm (2F, m); MS m/e = 210 (M+).

EXAMPLE 4

Epoxidation of a Mixture of "Allyl Telomers"

The oxidizing reagent (100 mmol) was prepared in 440 ml of $CH_3CN/H_2O$ solution (10:1) as in Example 1. Then 20 g of 1:1:1 mixture (molar) of $C_6F_{13}CH_2CH{=}CH_2$, $C_8F_{17}CH_2CH{=}CH_2$ and $C_{10}F_{21}CH_2CH{=}CH_2$ was dissolved in 50 ml of $CH_2Cl_2$ and added to the reagent solution. After 10 min the reaction was neutralized with bicarbonate. The reaction mixture was poured into water, extracted with $CFCl_3$ and worked-up as in Example 1. The 16.3 g of the respective epoxide mixture (1:1:1) obtained was practically pure.
MS

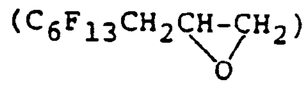

m/e = 360 (M+);

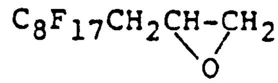

m/e = 460 (M+);

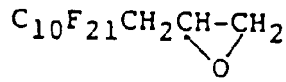

m/e = 560 (M+).

EXAMPLE 5

Epoxidation of a Diene

Using a procedure similar to that in Example 1, a solution of 240 mmoles of oxidizing reagent was prepared in 440 mL of acetonitrile/water (10:1) solution. To this solution was added 10 g of 3,3,4,4,5,5,6,6,7,7,8,8,9,9,10,10,11,11,12,12-eicosafluorotetradeca-1,13-diene dissolved in 30 mL of $CH_2Cl_2$. After standing overnight at room temperature the reaction was worked up in a similar manner to Example 1, by being neutralized with bicarbonate, poured into water, extracted with $CFCl_3$, and dried over anhydrous magnesium sulfate. The solvent was evaporated under reduced pressure. GC/MS analysis showed that both the mono- and bisepoxides were obtained in 30% and 55% yields, respectively. The reaction can be repeated on this crude product to increase the yield of the bisepoxide. $^1H$ NMR of the bisepoxide: 3.5 ppm (1H, 3 narrow m), 3.0 ppm (2H, m). MS, m/e (bisepoxide): 586 ($M^+$), 536 ($M^+$-$CF_2$), 486 ($M^+$-2$CF_2$), 100 $[(CF_2)_2]^+$. MS, m/e (monoepoxide) : 551 ($M^+$-F), 77 $(CF_2CH{=}CH_2)^+$.

EXAMPLE 6

Attempted Epoxidation of (Perfluorobutyl)ethylene by Trifluoroperacetic Acid

A mixture of 5.7 mL (0.1 mol) of 50% $H_2O_2$ and 50 mL of methylene chloride was stirred at 0-10°C while there was added dropwise 42.4 mL (63 g, 0.3 mol) of trifluoroacetic anhydride. The cold mixture was stirred for 10 min., then treated at 0°C with a solution of 24.6 g (0.10 mol) of (perfluorobutyl)ethylene in 50 mL of methylene chloride. No exotherm was discerned. The homogeneous mixture was refluxed for two hours while slow gas evolution occurred. Analysis of the cooled solution by GC showed $CF_3CF_2CF_2CF_2CH{=}CH_2$,

$CH_2Cl_2$ and $CF_3CO_2H$ to be present, but no epoxidized (perfluorobutyl)ethylene.

## Claims

1. A process for direct epoxidation of fluorine containing olefins comprising:
   A) generating an oxidizing reagent by passing fluorine through a mixture of acetonitrile and water; and
   B) contacting a fluorine containing olefin in a solvent with the oxidizing reagent to yield the desired epoxide; wherein the fluorine containing olefin comprises a compound of formula (I) or (II).

   $ZCH=CH_2$ (I)

   $YCH_2CH=CH_2$ (II)

   wherein
   Z is $ACF_2$-, or perfluoroaryl;
   Y is

   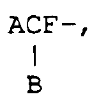

   or perfluoroaryl;
   A is perfluoroalkyl, fluorine, hydrocarbyl, or substituted hydrocarbyl; and
   B is fluorine or perfluoroalkyl.

2. The process of Claim 1 wherein the substituted hydrocarbyl group contains one or more of chlorine, fluorine, ester, ether, ketone, or vinyl groups.

3. The process of Claim 1 wherein Z is $ACF_2$-, Y is

   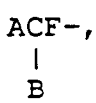

   and A is perfluorohydrocarbyl.

4. The process of Claim 3 wherein B is fluorine.

5. The process of Claim 3 wherein A is perfluoroalkyl.

6. The process of Claim 5 wherein A is perfluoro-n-alkyl.

7. The process of Claim 6 wherein B is fluorine.

8. The process of Claim 1 wherein the olefin is (perfluorohexyl)ethylene.

9. The process of Claim 1 wherein the olefin is (pentafluorophenyl)ethylene.

10. The process of Claim 1 wherein the olefin is (perfluorobutyl)ethylene.

11. The process of Claim 1 wherein the olefin comprises a mixture of allyl telomers.

12. The process of Claim 1 wherein the fluorine is diluted with nitrogen prior to contact with the mixture of acetonitrile and water.

13. The process of Claim 12 wherein the ratio of acetonitrile to water is about 10:1.

14. The process of Claim 13 wherein the mixture of acetonitrile and water is cooled to a temperature of about -10°C prior to contact with the fluorine.

15. The process of Claim 14 wherein the fluorine is passed at a rate of about 400 ml per minute through the mixture of acetonitrile and water.

16. The process of Claim 1 wherein the fluorine containing olefin is dissolved in a solvent selected from methylene chloride, chloroform, or a fluorocarbon.

17. The process of Claim 1 wherein the fluorine containing olefin is contacted with a 6-10 fold excess of the oxidizing reagent.

18. The process of Claim 17 wherein the epoxidation is conducted at a temperature of from about -15°C to 30°C.

19. The process of Claim 18 wherein the epoxidation is conducted at a temperature of from about 0°C to about 25°C.

20. The process of Claim 19 wherein the reaction time is from about 1 minute to about 3 hours.

21. The process of Claim 19 wherein the fluorine containing olefin comprises $ACF_2CH=CH_2$ wherein A is perfluoro-n-alkyl.

22. The process of Claim 19 wherein the fluorine containing olefin comprises

    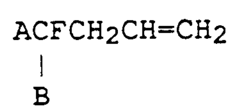

    wherein A is perfluoro-n-alkyl and B is fluorine.

**23.** The process of Claim 1 wherein Z and Y are independently perfluoroaryl.

**24.** The process of Claim 1 wherein Z is $ACF_2$-, A is $CH_2=CH(CF_2)_n$-, and n is an integer from 0 to about 30.

**25.** The process of Claim 19 wherein Z is $ACF_2$-, A is $CH_2=CH(CF_2)_n$-, and n is an integer from 0 to about 30.

**Patentansprüche**

**1.** Verfahren zur direkten Epoxidierung fluorhaltiger Olefine, umfassend

A) das Erzeugen eines oxidierenden Reagens' durch Hindurchleiten von Fluor durch eine Mischung aus Acetonitril und Wasser und

B) das In-Berührung-Bringen eines fluorhaltigen Olefins in einem Lösungsmittel mit dem oxidierenden Reagens zur Bildung des gewünschten Epoxids;

worin das fluorhaltige Olefin eine Verbindung der Formeln (I) oder (II)

$$ZCH=CH_2 \quad (I)$$

$$YCH_2CH=CH_2 \quad (II)$$

umfaßt, worin

Z ACF$_2$- oder Perfluoraryl ist;

Y

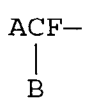

oder Perfluoraryl ist;

A Perfluoralkyl, Fluor, Hydrocarbyl oder substituertes Hydrocarbyl ist; und

B Fluor oder Perfluoralkyl ist.

**2.** Verfahren nach Anspruch 1, worin die substituierte Hydrocarbyl-Gruppe eine oder mehrere Chlor-, Fluor-, Ester-, Ether-, Keton- oder Vinyl-Gruppen enthält.

**3.** Verfahren nach Anspruch 1, worin Z $ACF_2$- ist, Y

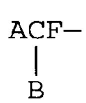

oder Perfluoraryl ist; und

A Perfluorhydrocarbyl ist.

**4.** Verfahren nach Anspruch 3, worin B Fluor ist.

**5.** Verfahren nach Anspruch 3, worin A Perfluoralkyl ist.

**6.** Verfahren nach Anspruch 5, worin A Perfluor-n-alkyl ist.

**7.** Verfahren nach Anspruch 6, worin B Fluor ist.

**8.** Verfahren nach Anspruch 1, worin das Olefin (Perfluorhexyl)ethylen ist.

**9.** Verfahren nach Anspruch 1, worin das Olefin (Perfluorphenyl)ethylen ist.

**10.** Verfahren nach Anspruch 1, worin das Olefin (Perfluorbutyl)ethylen ist.

**11.** Verfahren nach Anspruch 1, worin das Olefin eine Mischung aus Allyl-Telomeren ist.

**12.** Verfahren nach Anspruch 1, worin das Fluor mit Stickstoff verdünnt wird, bevor es mit der Mischung aus Acetonitril und Wasser in Berührung kommt.

**13.** Verfahren nach Anspruch 12, worin das Verhältnis Acetonitril zu Wasser etwa 10 : 1 beträgt.

**14.** Verfahren nach Anpruch 13, worin die Mischung aus Acetonitril und Wasser vor der Berührung mit dem Fluor auf eine Temperatur von etwa -10 °C gekühlt wird.

**15.** Verfahren nach Anspruch 14, worin das Fluor mit einer Rate von etwa 400 ml/min durch die Mischung aus Acetonitril und Wasser hindurchgeleitet wird.

**16.** Verfahren nach Anspruch 1, worin das fluorhaltige Olefin in einem Lösungsmittel gelöst wird, das aus Methylenchlorid, Chloroform oder einem Fluor-Kohlenstoff ausgewählt ist.

**17.** Verfahren nach Anspruch 1, worin das fluorhaltige Olefin mit einem 6- bis 10-fachen Überschuß des oxidierenden Reagens' in Berührung gebracht wird.

**18.** Verfahren nach Anspruch 17, worin die Epoxidierung bei einer Temperatur von etwa -15 °C bis 30 °C durchgeführt wird.

**19.** Verfahren nach Anspruch 18, worin die Epoxidierung bei einer Temperatur von etwa 0 °C bis 25 °C durchgeführt wird.

**20.** Verfahren nach Anspruch 19, worin die Reaktionszeit 1 min bis 3 h beträgt.

**21.** Verfahren nach Anspruch 19, worin das fluorhaltige Olefin $ACF_2CH=CH_2$ umfaßt, worin A Perfluor-n-alkyl ist.

**22.** Verfahren nach Anspruch 19, worin das fluorhaltige Olefin

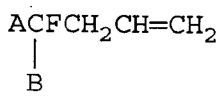

ist, worin A Perfluor-n-alkyl ist und B Fluor ist.

**23.** Verfahren nach Anspruch 1, worin Z und Y unabhängig voneinander Perfluoraryl sind.

**24.** Verfahren nach Anspruch 1, worin Z $ACF_2$- ist, A $CH_2=CH(CF_2)_n$- ist und n eine ganze Zahl von 0 bis etwa 30 ist.

**25.** Verfahren nach Anspruch 19, worin Z $ACF_2$- ist, A $CH_2=CH(CF_2)_n$- ist und n eine ganze Zahl von 0 bis etwa 30 ist.

**Revendications**

**1.** Un procédé d'époxydation directe d'oléfines fluorées comprenant les opérations consistant :

A) à générer un réactif oxydant en faisant passer du chlore sur un mélange d'acétonitrile et d'eau ; et

B) à mettre en contact une oléfine fluorée dans un solvant avec l'agent oxydant pour obtenir l'époxyde désiré, l'oléfine fluorée comprenant un composé répondant à l'une des formules (I) et (II) :

$ZCH=CH_2$ (I)

$YCH_2CH=CH_2$ (II)

où Z est un groupe $ACF_2$- ou perfluoroaryle et Y un groupe AC(B)F- ou perfluoroaryle, A étant un groupe perfluoroalkyle, un atome de fluor, un groupe hydrocarbyle ou un groupe hydrocarbyle substitué et B étant un atome de fluor ou un groupe perfluoroalkyle.

**2.** Le procédé de la revendication 1 dans lequel le groupe hydrocarbyle substitué renferme un ou plusieurs représentants de l'ensemble formé par les atomes de chlore et de fluor et les groupes esters, éthers, cétones et vinyle.

**3.** Le procédé de la revendication 1 dans lequel Z est un groupe $ACF_2$-, Y est un groupe AC(B)F- et A est un groupe perfluorohydrocarbyle.

**4.** Le procédé de la revendication 3 dans lequel B est l'atome de fluor.

**5.** Le procédé de la revendication 3 dans lequel A est un groupe perfluoroalkyle.

**6.** Le procédé de la revendication 5 dans lequel A est un groupe perfluoro-n-alkyle.

**7.** Le procédé de la revendication 6 dans lequel B est un atome de fluor.

**8.** Le procédé de la revendication 1 dans lequel l'oléfine est le (perfluorohexyl)éthylène.

**9.** Le procédé de la revendication 1 dans lequel l'oléfine est le (pentafluorophényl)éthylène.

**10.** Le procédé de la revendication 1 dans lequel l'oléfine est le (perfluorobutyl)éthylène.

**11.** Le procédé de la revendication 1 dans lequel l'oléfine comprend un mélange de télomères allyliques.

**12.** Le procédé de la revendication 1 dans lequel le fluor est dilué avec de l'azote avant mise en contact avec le mélange d'acétonitrile et d'eau.

**13.** Le procédé de la revendication 12 dans lequel le rapport de l'acétonitrile à l'eau est d'environ 10:1.

**14.** Le procédé de la revendication 13 dans lequel le mélange d'acétonitrile et d'eau est refroidi à une température d'environ -10°C avant d'être mis en contact avec le fluor.

**15.** Le procédé de la revendication 14 dans lequel on fait traverser au fluor le mélange d'acétonitrile et d'eau sous un débit d'environ 400 ml par minute.

**16.** Le procédé de la revendication 1 dans lequel l'oléfine fluorée est dissoute dans un solvant choisi parmi le chlorure de méthylène, le chloroforme et un fluorocarbone.

**17.** Le procédé de la revendication 1 dans lequel l'oléfine fluorée est mise en contact avec un excès de 6 à 10 fois du réactif oxydant.

**18.** Le procédé de la revendication 17 dans lequel l'époxydation est conduite à une température d'environ -15 à +30°C.

**19.** Le procédé de la revendication 18 dans lequel l'époxydation est conduite à une température d'environ 0 à 25°C.

**20.** Le procédé de la revendication 19 dans lequel le temps de réaction est d'environ 1 minute à environ 3 heures.

**21.** Le procédé de la revendication 19 dans lequel l'oléfine fluorée comprend $ACF_2CH=CH_2$, où A est un groupe perfluoro-n-alkyle.

**22.** Le procédé de la revendication 19 dans lequel l'oléfine fluorée comprend $AC(B)FCH_2CH=CH_2$, où A est un groupe perfluoro-n-alkyle et B l'atome de fluor.

**23.** Le procédé de la revendication 1 dans lequel Z et Y sont indépendamment des groupes perfluoroaryles.

**24.** Le procédé de la revendication 1 dans lequel Z est $ACF_2$-, A est $CH_2=CH(CF_2)n$- et n est un nombre entier de 0 à environ 30.

**25.** Le procédé de la revendication 19 dans lequel Z est $ACF_2$-, A est $CH_2=CH(CF_2)n$- et n est un nombre entier de 0 à environ 30.